# EUROPEAN PATENT SPECIFICATION

(11) **EP 1 492 878 B1**
(45) Date of publication and mention of the grant of the patent: **30.05.2007**
(21) Application number: 02716973.9
(22) Date of filing: 26.03.2002
(51) Int. Cl.: C12P 21/00, C07K 14/505, A61K 38/18, G01N 33/68

(54) **PROCESS FOR THE PREPARATION OF A DESIRED ERYTHROPOIETIN GLYCO-ISOFORM PROFILE**
VERFAHREN FÜR DIE HERSTELLUNG EINES GEWÜNSCHTEN PROFILS VON ERYTHROPOIETIN GLYKO-ISOFORMEN
PROCEDE DE PREPARATION DU PROFIL DE GLYCO-ISOFORMESD'ERYTHROPOIETINE RECHERCHE

(43) Date of publication of application: 05.01.2005
(73) Proprietor: Lek Pharmaceutical and Chemical Co. D.D., 1526 Ljubljana (SI)
(72) Inventor: SVETINA, Monica, 1000 Ljubljana (SI); SVETEK, Jelka, 1000 Ljubljana (SI); GANTAR-KSELA, Mateja, 1000 Ljubljana (SI); BRINC, Matjaz, 1000 Ljubljana (SI); FRANCKY, Andrej, 1000 Ljubljana (SI)
(74) Representative: Dietz, Jörg-Reimar
(86) International application number: PCT/IB2002/000971
(87) International publication number: WO 2003/080852

(56) References cited:
- EP-A- 0 984 062
- WO-A-96/35718
- WO-A-99/28346
- KRYSTAL G ET AL: "CM AFFI-GEL BLUE CHROMATOGRAPHY OF HUMAN URINE A SIMPLE 1-STEP PROCEDURE FOR OBTAINING ERYTHROPOIETIN SUITABLE FOR IN-VITRO ERYTHROPOIETIC PROGENITOR ASSAYS" BRITISH JOURNAL OF HAEMATOLOGY, vol. 58, no. 3, 1984, pages 533-546, XP008004465 ISSN: 0007-1048
- GOKANA A ET AL: "Chromatographic separation of recombinant human erythropoietin isoforms" JOURNAL OF CHROMATOGRAPHY A, ELSEVIER SCIENCE, NL, vol. 791, no. 1-2, 12 December 1997 (1997-12-12), pages 109-118, XP004107601 ISSN: 0021-9673 cited in the application

## Description

### BACKGROUND OF THE INVENTION

### Field of the invention

The invention relates to a new process for isolation of erythropoietin (EPO) by using a specific combination of chromatographic steps. This process enables the production of erythropoietin with high purity and with a desired profile of EPO glyco-isoforms.

EPO is a glycoprotein which plays a major role in the proliferation and differentiation of erythroid progenitor cells to erythrocytes. EPO obtained with recombinant DNA technology (recombinant EPO) is used for clinical application.

EPO exists as a mixture of glyco-isoforms, which differ in the number of charged carbohydrate moieties of the protein. The group of different mixtures of EPO glyco-isoforms comprises EPO-alpha, EPO-beta and EPO-omega.

### Description of the Prior Art

EPO and processes for its production are described, for example, in EP148605, EP205564 and EP255231.

EPO can be isolated from different sources, which also comprise genetically modified mammalian cells. Methods of isolation/purification which are known from the scientific and patent literature comprise different chromatographic steps. The most commonly used are anion exchange chromatography and reverse phase HPLC (RP-HPLC). Other chromatographic methods are also used: hydroxyapatite, hydrophobic, cation exchange, affinity (i.e. immunoaffinity) and size exclusion (gel filtration) chromatographies. Some intermediate steps are also common: salting out, concentration, diafiltration, ultrafiltration, dialysis, precipitation with ethanol and others.

Processes for the isolation of EPO are described in several European patents and patent applications and also in the scientific literature.

Methods for isolation of EPO, which comprise use of RP-HPLC, are described in EP205564, EP148605, EP830376, EP267678, EP228452, EP1127063, EP209539 and in Lai et al. (1986) J Biol Chem, 261(7):3116-21, Broudy et al. (1988) Arch Biochem Biophys, 265(2):329-336, Zou et al. (1998) Se Pu, Inoue et al (1994) Biol Pharm Bull, 17(2):180-4, Qian et al. (1986) Blood, 68(1):258-62, Krystal et al. (1986) Blood 67(1):71-9 and Lange et al (1984) Blood Cells, 10(2-3):305-14. RP-HPLC require in most cases the use of organic nonpolar solvents. The organic nonpolar solvents are toxic, pollute the environment and are difficult to separate from the desired proteins.

Methods for isolation of EPO, which comprise immunoaffinity chromatography with bound monoclonal antibodies, are described in: Sasaki et al. (1987) Methods Enzymol, Yanagawa et al. (1984) J Biol Chem, 259(5):2707-10 and Ghanem et al. (1994) Prep Biochem, 24(2):127-42. Monoclonal antibodies are mammalian proteins, their stability for large scale preparation is questionable, and cleaning in place and sanitations are difficult to be performed. There is also a risk of infection with viruses.

In EP358463 a combination of ion exchange chromatography and chromatography with bound lectin for the isolation of EPO is described. This process gives low yield only.

The use of lectin matrix in the combination with salting out and gel filtration is described in EP1010758. Again, a low yield is obtained.

The use of dihydroxyboronil matrix for the purification of EPO is described in EP820468. It results in a low purity which is not usable in human medicine.

The EP1127063 describes the isolation of EPO by using precipitation with ammonium sulfate, hydrophobic chromatography, anion and cation exchange chromatographies, gel filtration or different combinations of the described chromatographies. This process provides EPO which can be prepared in large scale with a high purity being suitable for use in human medicine. The use of ammonium sulfate can cause the problems with scalability, additional equipment is required and there is a need of changes of phases.

The EP984062 describes the isolation of EPO by using: chromatography with the matrix-bound stain, hydrophobic chromatography, hydroxyapatit chromatography and anion exchange chromatography. Neither the isolation of a desired profile of isoforms, nor the possibility of altering the EPO glyco isoforms with a combination of chromatographic step is described.

In EP640619, the isolation of specific EPO glyco-isoforms by using anion exchange chromatography is described. There is, however, no description about a process for preparation of mixture of different EPO glyco-isoforms. Furthermore, the process does not enable the isolation of EPO with high purity.

The EP428267 describes a process for the isolation of specific EPO glyco-isoforms which includes a preparative isoelectric focussing step, and a process for the isolation of mixtures of EPO glyco-isoforms with 12 or more sialic acids per molecule which includes an anion exchange chromatographic step performed after RP-HPLC purification steps. Furthermore, the process does not allow an aimed control of the desired mixture of isoforms, especially those with less than 12 sialic acids. Neither the isolation of desired profile of isoforms, nor the possibility of altering the EPO glyco isoforms with a combination of chromatographic step is described.

A process for isolation of exact number of EPO glyco-isoforms of an EPO analog is described in EP1037921. The process uses RP-HPLC.

The separation of specific EPO glyco-isoforms by using capillary isoelectring focusing is described in Cifuentes et al. (1999), J Chromatogr A, 15(2):453-63. However, urea is used in this process, which hampers clinical applications. The use of capillary isoelectrofocusing is not suitable for large scale preparation in industry.

The separation of EPO glyco-isoforms by a process which uses different chromatographic methods is described also in Gokana et al. (1997) J Chromat A, 791:109-18. The process includes the use of immunoaffinity chromatography with bound monoclonal antibodies. This increases the risk for possible viral infection which is detrimental for clinical applications, the stability for large scale preparation is questionable and the cleaning in place and sanitations are difficult to be performed. Furthermore, it includes the use of RP-HPLC which usually requires toxic organic solvents. It is therefore difficult to use this system for industrial application.

### SUMMARY OF THE INVENTION

It is an object of the invention to improve the process for producing EPO.

The present invention provides a. process for producing EPO according to claim 1 and the dependent claims.

The term 'EPO glyco-isoform' refers to EPO preparation having a single pl, and having the same amino acid sequence. Different EPO glyco-isoforms differ predominantly by the content of charged carbohydrate moieties (sialic acids) per EPO molecule.

The content of sialic acids contributes to the acidity of an EPO glyco-isoform and thus defines the position of each band in the isoelectric focusing (IEF) gel.

The numbering of band positions in the IEF gel is arbitrary. Larger numbers indicate increased acidity, i.e. higher content of sialic acids. Accordingly, EPO alpha (epoietin alfa - Eprex^{™}) contains EPO glyco-isoforms from 3 to 8, BRP standard (a reference standard of European Pharmacopoeia - Erythropoietin BRP Batch 1) contains EPO glyco-isoforms from 1 to 8, EPO beta (epoietin beta -Neorecormon^{™}) contains EPO glyco-isoforms from 1 to 8. BRP standard is an 50:50 blend of the two EPO preparations currently available on the European market (epoietin alfa and epoietin beta).

According to Lai et al. (1986) J Biol Chem and EP 428267 EPO alpha is described as a mixture of EPO glyco-isoforms containing 9 to 14 sialic acid groups per EPO molecule and these EPO glyco-isoforms can be correspondingly attributed to the arbitrary numbers 3 to 8 (used for the presentations of the EPO glyco-isoforms of this invention).

The essential element of the invention is the production of EPO with high purity and with a desired profile of EPO glyco-isoforms by using a combination of specific chromatographic steps in such a manner that the starting EPO glyco-isoform profile is changed or modified. The chromatographic steps applied in the process of the invention include at least (a) dye affinity chromatography, and (b) hydrophobic chromatography and (c) anion-exchange chromatography. In a preferred embodiment, the process of the invention further includes (d) gel filtration chromatography.

In the process for production of EPO according to the invention, the profile of EPO glyco-isoforms is changed or modified by using each of the above mentioned chromatographic steps. The performance of all chromatographic steps (a) to (c), preferably with (d) allows to obtain a more refined, predetermined EPO glyco-isoform profile. It is thus possible to control the profile of EPO glyco-isoforms during the process of isolation and therefore to obtain an exact and desired mixture of EPO glyco-isoforms (EPO alpha, EPO beta and others) with high purity.

The desired profile of EPO glyco-isoforms is suitably obtained by adjusting particular process factors, especially selecting appropriate matrixes for the above mentioned chromatographic steps, applying appropriate conditions in the washing/elution steps of the respective chromatographic methods and/or collecting appropriate fractions eluted from the chromatography columns.

The process of the present invention can advatageously be combined with a process for the analysis of the profile of EPO glyco-isoforms by subjecting samples containing EPO, which are obtained at selected intermediate steps during a multistep process of preparing EPO in the form of a glyco-isoform mixture, and optionally also at the beginning and/or the end of the whole process, to IEF with an appropriate gel matrix such as polyacrylamide, and then performing immuno-detection on a solid phase. That is, the analysis is performed as an intermittent in-process step in the course of the isolation and purification process, preferably at least twice. The immuno-detection can be performed on the basis of the Western-Blot technique on a suitable membrane such as a nitrocellulose membrane. By means of this process, an in-process control of samples during the process of purification is enabled. This process is therefore useful for research, optimisation procedures and the industrial application of the processes for the production of EPO with a specific and desired profile of EPO glyco-isoforms. When combined with the process for producing EPO having a desired EPO glyco-isoform profile of the invention as defined above, this in-process analysis allows an in line control at selected chromatographic steps, optionally before and after each step. It enables a control over the effectiveness of the desired EPO glyco-isoform profile change and the conditions to be adjusted at the purification steps in view of the desired EPO glyco-isoform profile. It therefore enables a targeted development and production of EPO. A profile-directed production of EPO can therefore be performed. A high and uniform product specificity and a high product quality can thereby be maintained.

The present invention enables large scale preparation (high quantities) of biologically active EPO with high purity and a desired profile of EPO glyco-isoforms. It is therefore suitable for industrial production of EPO.

With the present invention, several advantages can be obtained at the same time. The process of the invention provides a desired profile of EPO glyco-isoforms. The EPO purity is high by reaching a purity exceeding at least 99 % of total proteins and advantageously exceeding 99.9 % of total proteins, as determined by HPLC and gel electrophoresis. The process is suitable for large scale preparation of EPO. Since it is not necessary to carry out a RP-HPLC purification step, toxic non-polar organic solvents can be avoided. Furthermore, neither proteins nor other substances of animal origin are used. Therefore, the risk of contamination by viruses and the like and, hence, the risk of infections of patients is avoided, and the clinical safety is improved. The EPO obtained is suitable for use in human medicine.

### BRIEF DESCRIPTION OF THE DRAWINGS

Fig. 1 shows the separation of EPO glyco-isoforms in the samples of supernatants at the 6^{th}, the 10^{th} and the 11^{th} day of cultivating the CHO cells expressing EPO. The separation was performed by using IEF and immuno-detection on a membrane according to Example 1.
Fig. 2 shows the profiles of relative proportions of EPO glyco-isoforms in the samples deriving from CHO cell suspension cultures presented in Fig. 1.
Fig. 3 shows the profiles of relative proportions of EPO glyco-isoforms after elution from a dye affinity chromatography column depending on pH conditions used for the elution buffer in Example 2a.
Fig. 4 shows the electrophoretogram of EPO glyco-isoforms after IEF and immuno-detection of EPO on a membrane as obtained in Example 2b.
   Lane 1: sample after elution from dye affinity chromatography column (1. step of purification procedure) (Example 2a, buffer B, pH 7.0)
   Lane 2: cell culture supernatant (before the 1. step of purification procedure)
   Lane 3: sample after elution from hydrophobic chromatography column.
Fig. 5 shows the profiles of relative proportions of different EPO glyco-isoform fractions after the performance of hydrophobic chromatography with a gradient elution from the column as obtained in Example 2c.
Fig. 6 shows the electrophoretogram of EPO glyco-isoforms after the performance of anion exchange chromatography DEAE column obtained in Example 2d, as analysed by IEF and immuno-detection of EPO on a membrane.
   Lane 1: eluate from the DEAE column
   Lane 2: EPO BRP standard
Fig. 7 shows the profiles of relative proportions of different EPO glyco-isoform fractions after the performance of anion exchange chromatography with a gradient elution from DEAE column obtained in Example 2e.
Fig. 8 shows the profiles of relative proportions of different EPO glyco-isoform fractions after the performance of another anion exchange chromatography with a gradient elution from Source 15Q column obtained in Example 2f.
Fig.9 shows the profiles of relative proportions of different EPO glyco-isoform fractions after the performance of gel chromatography, followed by IEF analysis (Example 2g).
Fig. 10 shows the profiles of relative proportions of EPO glyco-isoforms which are present in pooled fractions after gradient elution from DEAE and Source 15 Q columns, compared with EPO standards. (Example 2h)
Fig. 11 shows the profiles of relative proportions of EPO glyco-isoforms after three consecutive chromatographic steps which involve dye affinity, hydrophobic and anion exchange chromatographic steps, as analysed with IEF. For comparison, EPO alpha and non-purified EPO from the cell culture supernatant from a bioreactor was analysed by IEF. In this particular case isoform 8 was not detected due to low amount of the material loaded on the gel.

### DESCRIPTION OF THE PREFERRED EMBODIMENTS

The invention relates to different ways of applying specific chromatographic methods. According to the basic concept of the invention, the process for preparing erythropoietin (EPO) in the form of a glyco-isoform mixture comprises subjecting an EPO containing composition to chromatographic steps of (a) dye affinity chromatography and (b) hydrophobic chromatography, and (c) anion exchange chromatography, wherein the EPO glyco-isoform profile is changed after the chromatographic steps relative to the starting EPO containing composition. The term EPO glyco-isoform profile according to the invention means a mixture of defined EPO glyco-isoforms which are present in various proportions in the total mixture. It was found that by means of these steps (a) and (b), it is possible to change the profile by reducing and preferably eliminating the proportion of EPO glyco-isoforms having a low number of, for example, up to 3, preferably up to 4 and particularly up to 5 (including 5), sialic acid groups per EPO molecule. Reducing or preferably eliminating the proportion of this low number EPO glyco-isoforms is beneficial for the obtained EPO product in terms of stability and biological activity.

The starting EPO containing composition, which is subjected to the chromatographic steps and thus for the change of the profile, is suitably a composition which is obtained from the supernatant of a cell culture or growth medium in which recombinant human EPO (rHuEPO) is expressed. rHuEPO is expressed from genetically transfected mammalian host cells, for example Chinese Hamster Ovary cells (CHO) or Baby Hamster Kidney cells (BHK)). The cells can be cultivated in roller bottles, spinner flasks or bioreactors. Cell growth and cultivation can be carried out by batch process or by continuous process. When a continuous process is performed, it may be carried out as a perfusion culture in a bioreactor and continuous harvesting for an appropriate time period. In order to remove cells and possibly other contaminating materials from the culture medium, the supernatant may be subjected to a precipitation step, for example using ammonium sulphate. Preferably the supernatant is subjected to filtration. The EPO containing solution or filtrate is then applied to the dye affinity column.

The dye affinity chromatography in step (a) is carried out with a suitable matrix-bound dye, preferably using a triazin dye. The particularly preferred matrix-bound dye is Cibachron 3G, and a suitable column material is Blue Sepharose 6 Fast Flow. A factor, by which the EPO glyco-isoform profile can be affected, is the pH of the elution buffer. The pH of the elution buffer is preferably adjusted to a range of 5.0 to 9.0, more preferably 6.0 to 8.0. Different types of elutions can be used, preferably linear (gradient) or step elution, most preferably step elution. Different salts can be used for elution, preferably NaCl and KCI, most preferably NaCl.

The hydrophobic chromatography in step (b) is carried out with a hydrophobic matrix. A suitable matrix material is Sepharose which is modified by hydrophobic groups such as alkyl groups, as exemplified by butyl, or aryl groups, as exemplified by phenyl. A good reduction or elimination of the proportion of EPO glyco-isoforms having a low number of sialic acid groups per EPO molecule can be obtained by the use of Source matrix (polystyrene divinyl benzene) materials having hydrophobic groups such as isopropyl, phenyl or ether, Toyopearl matrixes having hydrophobic groups such as phenyl, butyl and ether, preferably butyl modified matrix carrier such as Butyl Sepharose 4 Fast Flow. Different types of elutions can be used, preferably linear (gradient) or step elution, most preferably step elution

The desired change of the glyco-isoform profile is further improved by carrying out step (c) of anion exchange chromatography. By this measure, the profile is significantly changed to provide a more refined and controlled EPO glyco-isoform mixture as desired. A preferred anion exchange chromatography material is DEAE Sepharose such as DEAE Sepharose Fast Flow or Source 30Q (quaternary charged aminoethyl ligand) or Source 15Q (quaternary charged ammonium ligand), preferably Source 15Q.

When the process of the invention further comprises the chromatographic step of (d) gel chromatography, a further selection process with respect to the desired EPO glyco-isoform mixture can be realized. The EPO gyco-isoforms differs depending on the fraction pooled from the gel chromatography column. A preferred gel chromatography column material is Superdex 200 or Sephacryl S-200.

In the process for preparing EPO as described above, the chromatographic steps preferably consist of the following steps in the order as indicated:
(a), (b), (c) or (a), (c), (b), each optionally with (d).
The indicated orders of chromatographic steps are favourable in terms of both effective purification and desired EPO glyco-isoform adjustment. As intermitting steps, buffer exchange and especially filtration, ultrafiltration and/or diafiltration are preferably carried out. But there is no need to change the buffer between the steps (a) and (b). In particular, ultrafiltration and diafiltration steps are performed between steps (b) and (c), and a further ultrafiltration step is carried out between steps (c) and (d). Other, conventional purification methods can be applied in addition, but can also be omitted. In particular, chromatographic steps of HPLC and hydroxyapatite requiring the further use of organic solvents can be avoided. Applying steps (a) to (d) only, optionally supplemented by buffer exchange or filtration techniques, already enables the isolation of high quality EPO in high yield, high purity and a desired profile of EPO glyco-isoforms. The product can thus be directly used for clinical applications in human medicine.

In the preparation process of the invention, the change of the EPO glyco-isoform profile is directed to prepare a mixture of EPO glyco-isoforms having a defined number of sialic acid groups per EPO molecule. The desired EPO glyco-isoform mixture is suitably prepared and precisely controlled (i) by selecting appropriate matrixes, (ii) by adjusting the chromatographic conditions and/or (iii) by collecting the desired fractions eluted from the column in the above chromatographic steps (a) to (c), optionally with (d). In order to produce a uniform and well defined EPO glyco-isoform mixture which is of high biological activity and stability, the chromatographic steps are preferably adjusted in a manner that a specific content of sialic acid groups per EPO molecule that corresponds to the range of 6 to 14 is obtained. If desired, the prepared EPO glyco-isoform mixture can thus correspond to EPO alpha (number of sialic acid groups of 8 to 13) or EPO beta (6-13) or EPO with a number of sialic acid groups ranging from 7 to 13. Furthermore, the proportion of a specific EPO isoform or of all isoforms in a mixture can also be varied.

The conditions of the respective chromatographic steps described above can be adjusted by using a suitable chromatographic matrix, adjusting the chromatographic conditions and/or selecting the eluted fractions. Suitable chromatographic conditions in terms of pH range, salt concentration or gradient and temperature are shown in the following Tables.

### General ranges:

| Chromatography | PH range | Salt conc. range /gradient | Temp. range °C |
|---|---|---|---|
| dye affinity | 5-9 | 0-2.5 M | 2-20 |
| Hydrophobic | 6-8 | 2.5-0 M | 4-25 |
| anion exchange | 5-8 | 0-0.30 M | 4-25 |
| gel chrom. | 6-8 | 0.15-1 M | 2-20 |

### Preferred ranges:

| Chromatography | PH range | Salt conc. range /gradient | Temp. Range |
|---|---|---|---|
| dye affinity | 6.5 - 7.5 | 0-2.5 M | 2-6 |
| hydrophobic | 6.5 - 7.5 | 2.5-0 M | 18-23 |
| anion exchange | 6.5 - 7.5 | 0-0.30 M | 18-23 |
| gel chrom. | 7.2 - 7.5 | 0.15-0.5 M | 2-6 |

The obtained EPO product having the desired glyco-isoform mixture is suitable for the manufacture of a pharmaceutical composition which comprises a therapeutically effective amount of EPO. EPO as obtained by the process of the invention is mixed with a conventional pharmaceutically acceptable carrier. A preferred pharmaceutical composition comprises EPO, buffer, polysorbates, different amino acids, optionally sugars and salts.

The process of the present invention is preferably combined with a process for the determination of erythropoietin (EPO) glyco-isoform profile in an EPO containing composition, the process comprising the steps of:
- providing an EPO containing composition from the culture supernatant, and /or at an intermediate step or at the end of an EPO isolation and purification process which involves multiple chromatographic steps, and/or from a pharmaceutical composition,
- subjecting the EPO containing composition to isoelectric focusing (IEF) in a gel matrix
- transfer of proteins from the gel to a membrane
- immuno-detection of EPO on the membrane.

A suitable gel matrix used for IEF is polyacrylamide. The transfer from the gel to a membrane, which is preferably a nitrocellulose membrane, can be performed by simple diffusion transfer or electro-blotting. The immuno-detection is suitably carried out by using EPO specific antibodies and appropriately labeled secondary antibodies. The use of IEF combined with the immuno-detection specific for EPO on the membrane enables the reliable analysis of the profile of EPO glyco-isoforms in the samples of mammalian cell cultures and in samples after each chromatographic step of the present invention as described above. This process enables to control the samples during the process of purification, that is an in-process control. It can thus be used by research, optimisation procedures and industrial application of the processes for the production of EPO with specific profile of EPO glyco-isoforms. The profile of EPO glyco-isoforms can be determined in the concentrated samples of purified EPO, in complex mixtures of proteins (cell cultures) and in solutions with low concentration of EPO and after particular chromatographic step and in all samples or solutions, which comprise other proteins. The analysis is specific and sensitive to EPO and some EPO analogs. The determined EPO glyco-isoform profile enables a direct comparison of EPO glyco-isoform compositions and quantitative proportions in different samples originating either from cell culture or from different eluats of the chromatographic steps. The process of determining the EPO glyco-isoform profile is preferably applied as an in-process control during the process of preparing erythropoietin (EPO) of the invention as described above.

The following examples are given for the purpose of illustrating various embodiments of the invention and are not meant to limit the present invention in any fashion.

### Example 1

IEF in polyacrylamide gel and immuno-detection on nitrocellulose membrane

This process was used for the analysis of the profile of EPO glyco-isoforms. The profile of EPO glyco-isoforms could be determined in different samples (in complex mixtures of proteins, in the samples with low concentrations of EPO). The analysis result enabled the direct comparison of EPO quality in different samples. Present Example 1 shows the IEF analysis and immuno-detection of EPO from cell cultures, and the subsequent Examples 2 and 3 show the results from eluates after a particular chromatographic step of the isolation and/or purification of EPO.

For a sample to be prepared for IEF analysis, it was subjected to diafiltration by using an ultrafiltration membrane (MwCO (molecular weight cut off) 10000 Da). The diafiltration step eliminates molecules smaller than 10 kDa and leads to a desalting and concentration of the sample. The final concentration of the sample is 0.5-1.5 µg EPO in 15 - 20 µl of the sample which is loaded in the gel.

Preparation of polyacrylamide IEF gel: the total concentration of acrylamide was 5 %, the degree of crosslinking was 3%, thickness was 0,5 mm, and final concentrations of urea and total ampholites were 5 M and 2%, respectively. The role of ampholites is to get a pH gradient from pH 3 to 6 in the gel (for example: 70% of total ampholites: pH 3-5; 30% of total ampholites pH 3-10). Electrode solutions were adapted to the choice of ampholites, for example the electrolyte on anode consisted of 0.1 M glutamic acid and 0.5 M phosphoric acid, the electrolyte on cathode was 0.1 M solution of β-alanine.

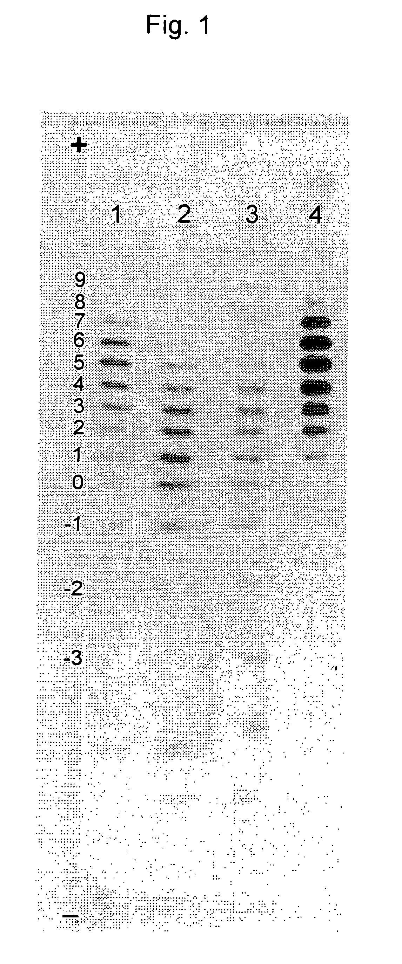

As shown in Figure 1, EPO glyco-isoforms in the samples of supernatants of cell cultures were separated by using IEF. In lanes 1-3, samples of EPO producing cell culture suspension (CHO cells) were taken at the 6^{th} day (lane 1), at the 10^{th} day (lane 2) and at the 11^{th} day (lane 3) from the beginning of cultivation and loaded on the gel. In lane 4, EPO BRP Batch 1 was loaded on the gel.

Prefocusing: 25 min at constant power P = 12 W (for the gel size of 25 x 11 cm). Samples (15 -20 µl) were loaded close to the cathode.

Focusing: 75 min at constant power P = 15 W (for the gel size of 25 x 11 cm). During the electrophoresis the gel was kept at 15 °C. The conditions for electrophoresis may be changed taking into account different characteristics of ampholites from different sources.

Diffusion transfer of proteins from the gel to the membrane was performed in the following way: the membrane was placed on the gel and several layers of filter paper were placed on the membrane. Gel, membrane and the filter paper were of the same size. Membrane and filter paper were prewetted in transfer buffer.
Transfer buffer: 48 mM Tris-HCl, 39 mM glycine, 0.037% SDS, 20 vol% methanol, pH 8,3. The transfer time is 90 min at 40 °C.

### Immuno detection on nitrocellulose membrane:

After transfer, the nitrocellulose was blocked using a 5% solution of skimmed powder milk blocking solution. Then, the membrane was incubated with mouse monoclonal anti hEPO antibodies (IgG) as primary antibodies. The membrane was washed by using 10 mMTris-HCl, 150 mM NaCl, pH 7.4. Then, the membrane was incubated with rabbit polyclonal antibodies (IgG) conjugated with horse raddish peroxidase as secondary antibodies
For the staining reaction, a substrate for peroxidase was used, for example 4-chloro-1-naphtol.
Specific EPO glyco-isoforms were identified by comparing with EPO BRP. Intensity of bands was determined by using densitometry. The profile of specific EPO glyco-isoforms was then calculated.

The electrophoretogram obtained by this process is shown in Fig. 1.

Separate bands seen on electrophoretogram are marked with arbitrary numbers from 3 to 9. They are marked according to the increasing acidity of the isoform from the negative cathode to the positive anode (higher numbers indicate isoforms with more sialic acids and therefore lower pl). In Fig. 1 and the subsequent Figures, the correlation between the arbitrary numbers in the electrophoretogram and the number of sialic acids groups per EPO molecule is as follows: isoforms 9,8,7,6..... are atributed to 14,13,12,11...... sialic acid groups per molecule, respectively.

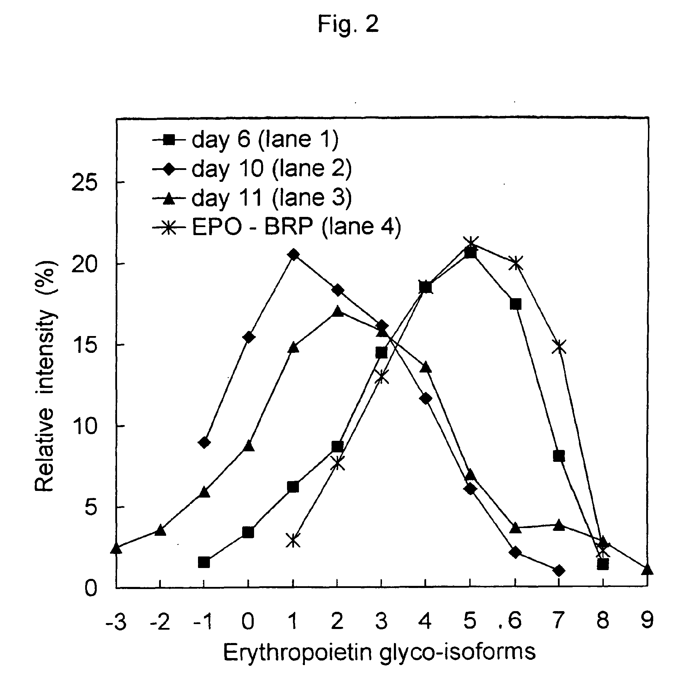

Fig. 2 shows the profiles of relative proportions of EPO glyco-isoforms in the samples derived from CHO cells suspension culture presented on Fig. 1. Intensity of bands on the membrane (Fig. 1) is measured by using densitometry. The intensity of each band is given as a relative part (relative intensity, %) of the sum of intensities of all detected bands for each sample (lane).

Figs. 1 and 2 show that the IEF analysis combined with immuno-detection according to the invention allows a good and specific control over the EPO glyco-isoform production from crude cell culture where many other proteins are present. By this analysis, an appropriate time point for harvesting can be found which provides a good basis for further isolation and purification in terms of the aimed glyco-isoform profile.

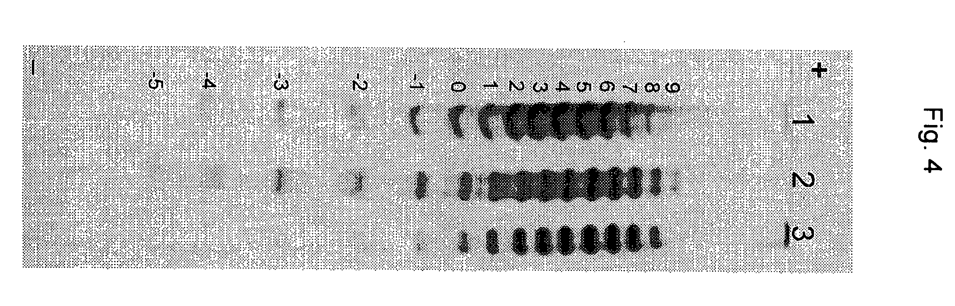

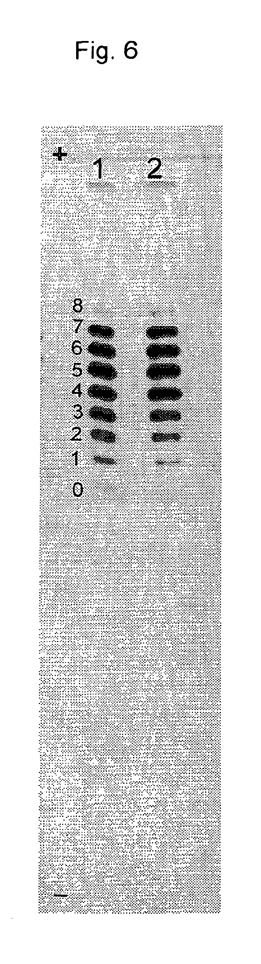

Figs. 4 and 6 show further electrophoretograms for EPO glyco-isoform analysis during the process of preparing EPO glyco-isoform mixture according to the invention (see Examples 2b and 2d, respectively).

### Example 2

Changing EPO glyco-isoform profiles by different chromatographic steps

### Example 2a: Cromatography on matrix - bound stain Cibachron Blue 3G

EPO producing CHO cell culture suspension was prepared in bioreactor, filtered first through a 10 µm prefilter and than through a 0.2 µm membrane sterilizing filter to separate the cells. The filtrate was loaded on the first chromatographic column by using matrix - bound stain Cibachron Blue 3G. The chromatography was performed under following conditions:

| | |
|---|---|
| Column | matrix Blue Sepharose 6 Fast Flow, Amersham Pharmacia Biotech; particle size 45 do 165 µm; |
| | CV (column volume) =7,85 ml, H (column height) = 10 cm, D (column diameter) = 1 cm |
| temp. | room temperature |
| Flow | 1,5 ml/min; 115 cm/hour |
| buffer A | 10 mM Na-phosphate, pH = 7,0 |
| buffer B | 10 mM Na-phosphate, 2,5M NaCl, pH=7,0 |
| Sample | 150 ml; 6 mg |

The column was equilibrated with 5 CV (column volume) of buffer A. After loading of the sample, the column was first washed with 3 CV of buffer A and then with 5 CV of mixture of buffer A and B (90:10). Most of EPO was eluted with buffer B (5 CV). The same separation was performed by using buffers A and B at pH 6.4 and 8.0.

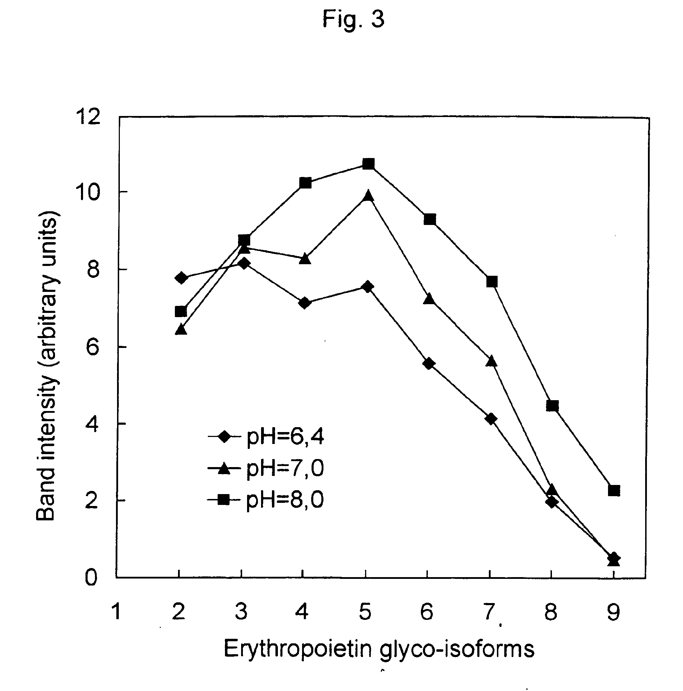

Pooled fractions after elution with buffer B were analysed by using IEF in polyacrylamide gel, transferring proteins to nitrocellulose membrane, and performing immuno-detection as described in Example 1. The intensity of bands on the membrane was measured densitometrically. The intensity of each band is given as a relative part (relative intensity, %) of the sum of intensities of all detected bands for each sample (lane). The separate bands seen on electrophoretogram are marked with arbitrary numbers from -3 to 9 (with the correlation between the indicated numbers and the content of sialic acids per EPO molecule as defined in Example 1).
The analysis showed that different profiles of EPO glyco-isoforms could be obtained by using different pH of washing and elution buffers as shown in Figure 3.

### Example 2b: Hydrophobic chromatography (1)

The eluate from Blue Sepharose 6 Fast Flow column (buffer B at pH 7) having a desired glyco-isoform profile was loaded on Butyl Sepharose 4 Fast flow (Amersham Pharmacia Biotech) column. The hydrophobic chromatography was performed under following conditions:

| | |
|---|---|
| Column | matrix Butyl Sepharose 4 Fast Flow, Amersham Pharmacia Biotech; average particle size 90 µm; |
| | CV = 1 ml, H=2,5cm, D = 0,7 cm |
| temp. | room temperature |
| Flow | 1 ml/min 150 cm/hour |
| buffer A | 10 mM Na-phosphate, 2,5 M NaCl, pH = 7,0 |
| buffer B | 10 mM Na-phosphate, 1,0 M NaCl, 30% (VN) isopropanol, pH=7, 0 |
| buffer C | 10 mM Na-phosphate, 30% (V/V) isopropanol, pH=7,0 |
| Sample | 1,5 ml, 0,9 mg |

The column was equilibrated with buffer A. After loading of the sample, the column was washed with 3 CV of buffer A and then with 8 CV of buffer B. Most of EPO was eluted with buffer C. Profile of EPO glyco-isoforms, which was obtained by washing the column with buffer C, was analysed with IEF in polyacrylamide gel and immuno-detection of EPO on nitrocellulose membrane, as shown in Fig. 4.
Lane 1: sample after elution from Blue Sepharose Fast Flow chromatography column (1. step of purification procedure) (Example 2a, buffer B, pH 7)
Lane 2: cell culture supernatant (before the 1. step of purification procedure)
Lane 3: sample after elution from Butyl Sepharose 4 Fast Flow column (pooled fractions after elution with buffer C).

IEF and immuno detection analysis was performed and evaluated as in Example 1.

As becomes apparent from Fig. 4, such chromatographic conditions were established that most of the EPO glyco-isoforms with lower content of sialic acids were eliminated.

The pooled fractions were concentrated by using the system for ultrafiltration MINITAN (Millipore). The dialfiltration was performed by using 10 mM Na-phosphate, pH 7.0 as buffer.

### Example 2c: Hydrophobic chromatography (2)

The eluate from Blue Sepharose 6 Fast Flow column (buffer B at pH 7) having a desired glyco-isoform profile was loaded on Butyl Sepharose 4 Fast flow (Amersham Pharmacia Biotech) column. The hydrophobic chromatography was performed under following conditions:

| | |
|---|---|
| Column | matrix Butyl Sepharose Fast Flow, Amersham Pharmacia Biotech; average particle size 90 µm; |
| | CV = 1 ml, H = 2,5 cm, D = 0,7 cm |
| temp. | Room temperature |
| Flow | 1 ml/min; 150 cm/hour |
| buffer A | 10 mM Na-phosphate buffer, 2,5 M NaCl, pH = 7,0 |
| buffer C | 10 mM Na-phosphate buffer, 30% (V/V) isopropanol, pH=7,0 |
| Sample | EPO BRP standard dissolved in buffer A; 0,5 ml; 0,25 mg |

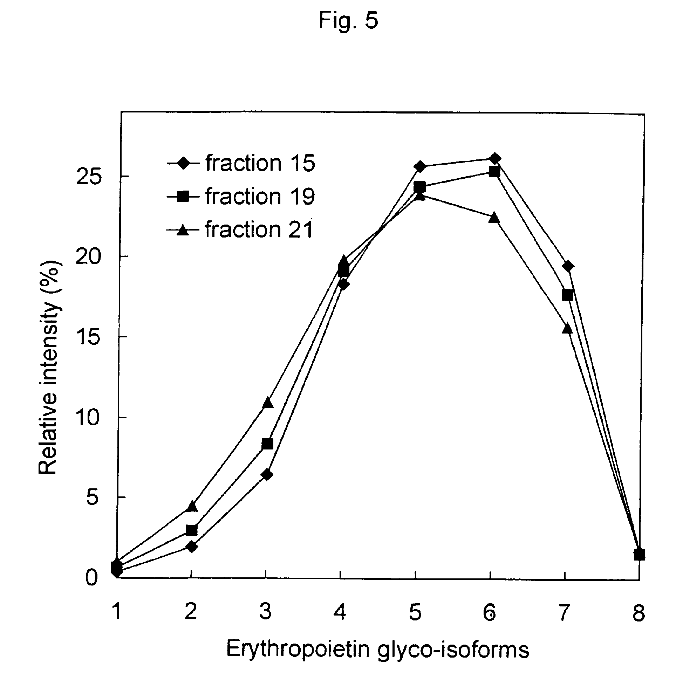

The column was equilibrated with buffer A. After loading the sample the column was washed with 3 CV of buffer A and then with 5 CV of mixture of buffers A and C (50:50). EPO was eluted from the column with linear gradient of buffer C in buffer A (linear gradient from 50 to 100 % of buffer C in 45 minutes (=45 CV)). Three overlapping peaks can be seen on the chromatogram. EPO was eluted in fractions 15 through 23. Fractions 15, 19 and 21 were then analysed by using IEF and immuno detection as described in Example 1. The results shown in Fig. 5 indicate that the profile of EPO glyco-isoforms differed from fraction to fraction.

EPO glyco-isoforms with lower pl (higher content of sialic acids) are eluted in earlier fractions than those with higher pl. This feature indicates that this method enables to collect the fraction(s) with desired profile of EPO glyco-isoforms.

### Example 2d: Anion exchange chromatography (1)

The eluate from Butyl Sepharose 4 Fast Flow column as described in Example 2c having a desired glyco-isoform profile was loaded on DEAE Sepharose Fast Flow (Amersham Pharmacia Biotech) column. The anion exchange chromatography was performed under following conditions:

| | |
|---|---|
| Sample | 0,2 mg |
| Column | matrix DEAE Sepharose Fast Flow, Amersham Pharmacia Biotech; |
| | average particle size 90 µm; |
| | CV = 1 ml; H = 2,5 cm, D = 0,7 cm |
| Flow | 1 ml/min; 158 cm/hour |
| buffer A | 10 mM Na-phosphate pH=7,0 |
| buffer B | 10 mM Na-phosphate pH=7,0; 0,03 M NaCl |
| buffer C | 10 mM Na-phosphate pH=7,0; 0,1 M NaCl |
| buffer D | 10 mM Na-phosphate pH=7.0; 0.3 M NaCl |

The column was equilibrated with buffer A. After loading of the sample, the column was washed with 3 CV buffer A and then with 5 CV of buffer B. Most of EPO was eluted with buffer C (8 CV). Remaining proteins were eluted with buffer D. The column was regenerated with 2 M NaCl.

The profile of EPO glyco-isoforms obtained by washing the column with buffer C was analysed with IEF and immuno-detection of EPO on nitrocellulose membrane as described in Example 1. The results are shown in Fig. 6.
Lane 1: eluate from DEAE Sepharose Fast Flow (pooled fractions after elution with buffer C)
Lane 2: EPO BRP Batch 1.

The described conditions enabled obtaining a profile which was essentially similar to the profile of EPO BRP standard.

### Example 2e: Anion exchange chromatography (2)

The eluate from Butyl Sepharose Fast Flow column as described in Example 2c having a desired glyco-isoform profile was loaded on DEAE Sepharose Fast Flow (Amersham Pharmacia Biotech) column. The anion exchange chromatography was performed under following conditions:

| | |
|---|---|
| Column | matrix DEAE Sepharose Fast Flow, Amersham Pharmacia Biotech; |
| | average particle size 90 µm; |
| | CV=1ml; H=2,5 cm, D = 0,7 cm |
| temp. | room temperature |
| Flow | 1 ml/min; 150 cm/hour |
| buffer A | 10 mM Na-phosphate pH=7,0 |
| buffer B | 10 mM Na-phosphate pH=7,0; 1 M NaCl |
| Sample | EPO BRP standard dissolved in buffer A; 0,5 ml; 0,25 mg |

The column was equilibrated with buffer A. After loading the sample, the column was washed with 3 CV of buffer A and then with 5 CV of mixture of buffer A and B (94:6). EPO was eluted from the column with continuously increasing the proportion of buffer B in buffer A (linear gradient from 6 to 11.5% of buffer B in 60 min (=60 CV)). Three overlapping peaks were seen on the chromatogram. EPO was eluted in fractions 8 through 24.

Fractions 11, 14, pooled fractions 21 and 22 (21 +22) and 24 were analysed by using IEF. Proteins were transferred to nitrocellulose membrane, and EPO was immuno-detected and the intensity of bands was measured densitometrically as described in Example 1.

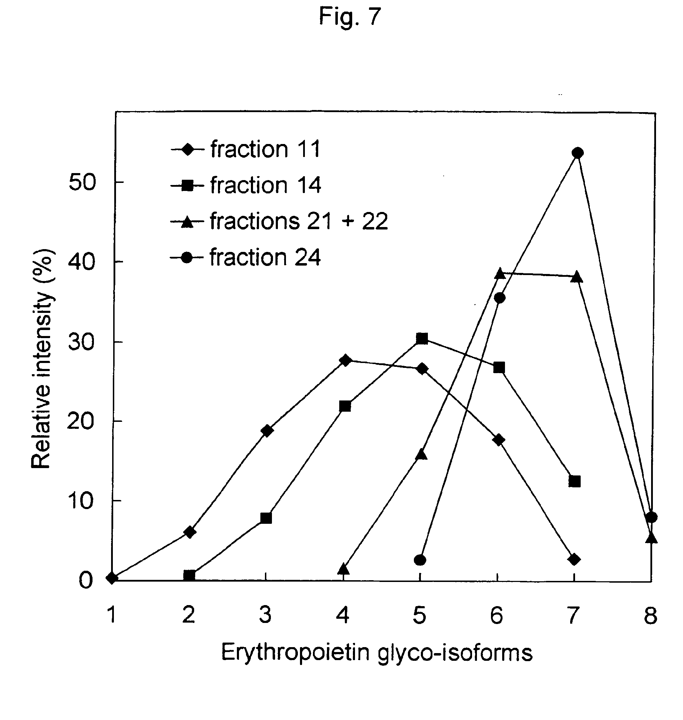

IEF analysis showed that the profile of EPO glyco-isoforms differed from fraction to fraction (Figure 7). EPO glyco-isoforms with higher pl (with less sialic acids) are eluted faster from the column. Therefore the method can be used for production of desired profile of EPO glyco-isoforms.

### Example 2f: Anion exchange chromatography (3)

The eluate from Butyl Sepharose Fast Flow column as described in Example 2c having a desired glyco-isoform profile was loaded on SOURCE 15Q (Amersham Pharmacia Biotech) column. The anion exchange chromatography was performed under following conditions:

| | |
|---|---|
| Column | matrix SOURCE 15Q, Amersham Pharmacia Biotech; particle size 15 µm |
| | CV = 1 ml, H=3cm, D=0,64cm |
| temp. | room temperature |
| Flow | 1 ml/min; 180 cm/hour |
| buffer A | 10 mM Na-phosphate pH=7,0 |
| buffer B | 10 mM Na-phosphate pH=7,0; 1 M NaCl |
| Sample | EPO BRP standard dissolved in buffer A; 0.5 ml; 0.25 mg |

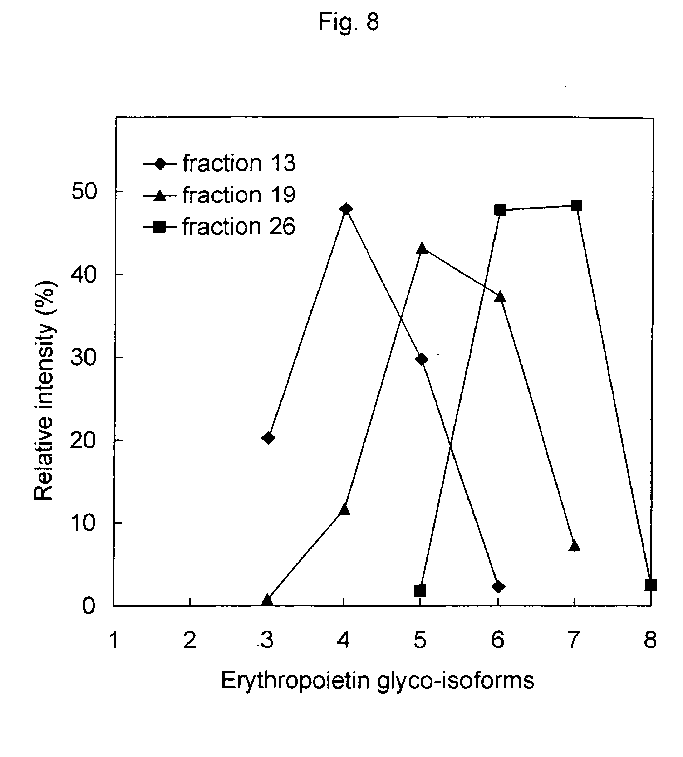

The column was equilibrated with buffer A. After loading the column was washed with 3 CV of buffer A and then with 5 CV of the mixture of buffers A and B (94:6). EPO was eluted from the column with continuously increasing the proportion of buffer B in buffer A (linear gradient from 6 to 11.5% of buffer B in 60 min (=60 CV)). Three overlapping profiles are seen on the chromatogram. EPO was eluted in fractions from 8 to 31. IEF analysis showed that the profile of EPO glyco-isoforms differed from fraction to fraction (Figure 8). Fractions 13, 19 and 26 were analysed with IEF, proteins were then transferred to the nitrocellulose membrane, and EPO was immunodetected as described in Example 1.

EPO glyco-isoforms with higher pl (lower content of sialic acids) are eluted faster from the column. Therefore the method can be used for isolation of desired profile of EPO glyco-isoforms.

### Example 2g: Gel filtration

The eluate from DEAE Sepharose Fast Flow (Amersham Pharmacia Biotech) column as described in Example 2d was loaded on Superdex 200 (Amersham Pharmacia Biotech) column. The gel filtration was performed under following conditions:

| | |
|---|---|
| Sample | 0.09 mg; 0.5 ml |
| Column | matrix Superdex 200, Amersham Pharmacia Biotech; average particle size 13 µm |
| | CV = 24 ml, H = 30 cm, D = 1 cm |
| Flow | 0,2 ml/min; 15,2 cm/hour |
| buffer A | 10 mM Na-phosphate, 0,15 M NaCl pH=7,2 |

EPO was eluted from the column in fractions 59 through 68.

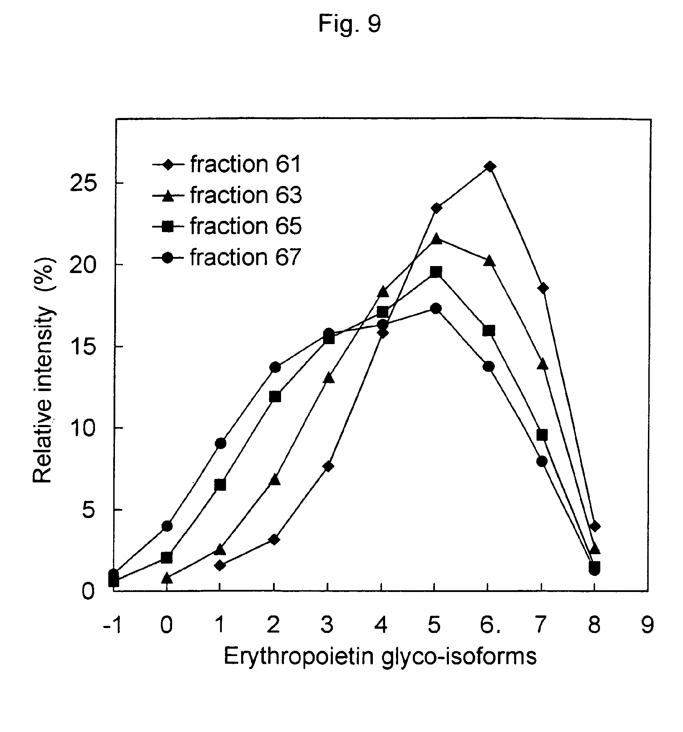

Fig. 9 shows the profiles of EPO glyco-isoforms after elution from the Superdex 200 column. Fractions 61, 63, 65 and 67 were analysed with IEF, proteins were then transferred to nitrocellulose membrane, the EPO was immuno-detected and the intensity of bands was measured densitometrically as described in Example 1.

The IEF analysis demonstrated that EPO glyco-isoforms differed between particular fractions. EPO glyco-isoforms with lower pl (with higher content of sialic acids) are eluted faster from the column. Therefore this method enables the isolation of a desired profile of EPO glyco-isoform by choosing the appropriate fraction after elution from the column.

### Example 2 h: Preparation of a desired EPO glyco-isoform mixture

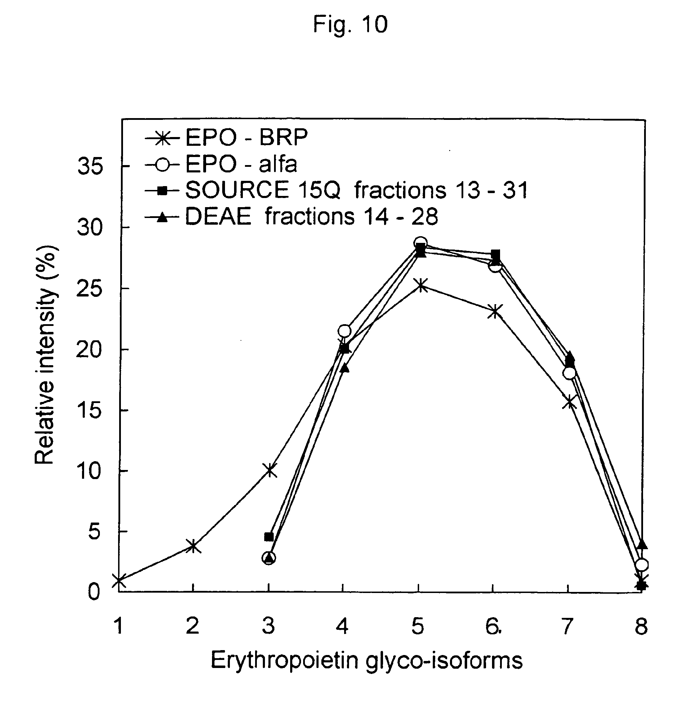

Figure 10 shows profiles of EPO glyco-isoforms after gradient elution from columns DEAE Sepharose Fast Flow of Example 2e and Source 15 Q of Example 2f. EPO BRP Batch 1 was loaded on the columns. Pooled fractions 13 through 31 from Source Q and pooled fractions from 14 to 28 from DEAE Sepharose Fast Flow were analysed by using IEF. For comparison EPO BRP and EPO alpha (Eprex) were also analysed. Proteins were then transferred to the nitrocellulose membrane, EPO was immunodetected and the intensity of bands was measured densitometrically .as described in Example 1. A good correspondence with the desired EPO alpha isoform profile is obtained with both anion exchange chromatography columns.

### Example 3

Process for obtaining a desired profile of EPO glyco-isoforms: profile of EPO alpha.

EPO producing CHO cell culture suspension was prepared in bioreactor, filtered first through 10 µm prefilter and than through 0.2 µm membrane sterilizing filter to separate the cells. The filtrate was loaded on the first chromatographic column with matrix - bound stain Cibachron Blue 3G. The chromatography was performed under following conditions:

| | |
|---|---|
| Column | matrix Blue Sepharose 6 Fast Flow, Amersham Pharmacia Biotech |
| | particle size: 45 -165 µm; |
| | column volume (CV) =30 ml, column length (H) = 15 cm |
| | column diameter (D) = 1.6 cm |
| temp. | Room temperature |
| flow | 5 ml/min; 150 cm/hour |
| buffer A | 10 mM Na-phosphate, pH = 7,0 |
| buffer B | 10 mM Na-phosphate, 2,5 M NaCl, pH=7,0 |
| Sample | 400 ml; 36 mg protein |

The column was equilibrated with 5 CV of buffer A. After loading of the sample, the column was washed with 5 CV of buffer A and then with 4 CV mixture of buffer A and buffer B (92:8). The eluate in which EPO was eluted by washing the column with buffer B (6 CV) was used for further the subsequent chromatographic steps.

The eluate from the first chromatographic step was loaded to the column of the second chromatographic step (hydrophobic chromatography). Hydrophobic chromatography was performed under following conditions:

| | |
|---|---|
| Column | Matrix Butyl Sepharose Fast Flow, Amersham Pharmacia Biotech; average particle size 90 µm; |
| | CV = 1 ml, H=2,5 cm, D = 0,7 cm |
| temp. | Room temperature |
| Flow | 1 ml/min 150 cm/hour |
| buffer A | 10 mM Na-phosphate, 2,5 M NaCl, pH = 7,0 |
| buffer B | 10 mM Na-phosphate, 30% (VN) isopropanole, pH=7,0 |
| Sample | 90 ml, 1,5 mg |

The column was equilibrated with 5 CV of buffer A. After loading the sample the column was washed with 5 CV of buffer A and then with 12 CV of mixture of buffers A and B (1:1). The eluate in which EPO was eluted with buffer B (15 CV) was used for the subsequent chromatographic steps.

The eluate from the second chromatographic step was concentrated and the buffer B was replaced with 10 mM Na-phosphate buffer, pH 7.0 by using the system for ultrafiltration Centricon YM-10 (Millipore). The concentrate was loaded to the column of the third chromatographic step (anion exchange chromatography). The chromatography was performed under following conditions:

| | |
|---|---|
| Column | matrix SOURCE 15Q, Amersham Pharmacia Biotech; particle size 15 µm |
| | CV = 1 ml, H=3cm, D=0,64cm |
| temp. | Room temperature |
| flow | 1 ml/min; 180 cm/hour |
| buffer A | 10 mM Na-phosphate pH=7,0 |
| buffer B | 10 mM Na-phosphate pH=7,0; 1 M NaCl |
| Sample | 2 ml, 0,4 mg |

The column was equilibrated with 5 CV of buffer A. After loading the sample, the column was washed with 5 CV of buffer A. EPO was eluted with linear gradient of buffer B in buffer A (linear gradient from 0 to 13% of buffer B in 60 min (= 60 CV). EPO was eluted in fractions from 20 to 56, while the desired glyco-isoform profile was found in fractions 37 to 51.

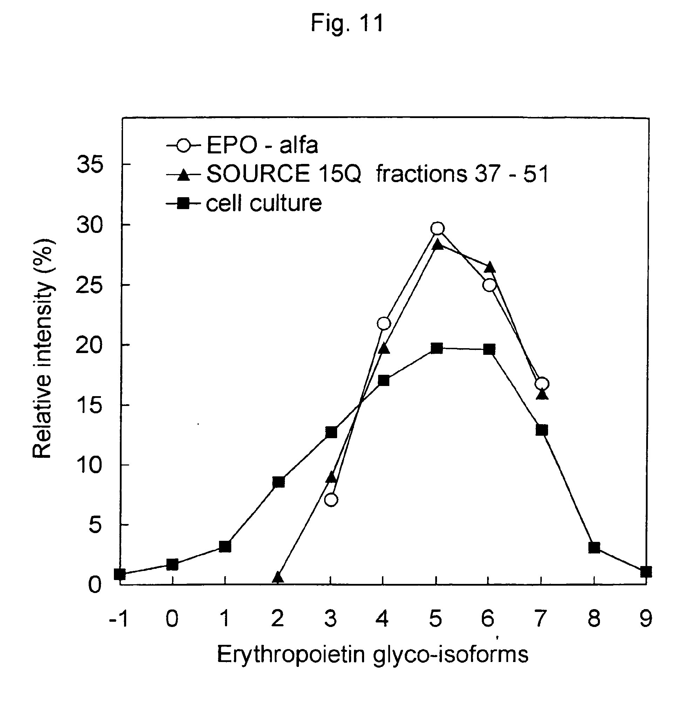

The final profile of glyco-isoforms of isolated EPO after three consecutive chromatographic steps using Blue Sepharose 6 Fast Flow, Butyl Sepharose 4 Fast Flow and Source 15Q is shown in Figure 11.
Pooled fractions 37 through 51 from Source 15Q were analysed with IEF as described in Example 1. For comparison two other samples were loaded: EPO alpha (Eprex) and non-purified supernatant from cell culture in bioreactor. As apparent from Fig. 11, an excellent correspondence between the EPO glyco-isoform profile and EPO alpha as desired was obtained by using the three characteristic chromatographic steps only. At the same time, the purity of the obtained EPO product was over 99 %, determined by HPLC and gel electrophoresis.

## Claims

1. A process for the preparation of erythropoietin which process comprises subjecting an erythropoietin containing composition to chromatographic steps of:
(a) dye affinity chromatography,
(b) hydrophobic chromatography and
(c) anion exchange chromatography,
**characterized in that** the chromatographic steps consist only of the following steps in the order as indicated:
(a), (b), (c) or (a), (c) (b), each optionally with
(d) gel chromatography.

2. The process according to claim 1, additionally comprising at least one intermittent step of buffer exchange, filtration, ultrafiltation and/or diafiltration.

3. The process according to any one of claim 1 or 2, wherein erythropoietin is prepared in the form of a glyco-isoform mixture.

4. The process according to claim 3, wherein the glyco-isoform profile of the prepared erythropoietin is changed after the chromatographic steps relative to the starting erythropoietin containing composition.

5. The process according to any of the preceding claims, wherein the dye affinity chromatography is carried out with a matrix-bound triazin dye.

6. The process according to any of the preceding claims, wherein in the dye affinity chromatography step the pH of the elution buffer is adjusted to a range of 5.0 to 9.0.

7. The process according to any of the preceding claims, wherein in the dye affinity chromatography step the pH of the elution buffer is adjusted to a range of 6.0 to 8.0.

8. The process according to any of the preceding claims, wherein the hydrophobic chromatography is carried out with a butylated matrix carrier.

9. The process according to any of the preceding claims, wherein the anion exchange chromatography is carried out on a DEAE Sepharose matrix.

10. The process according to any of the preceding claims wherein the gel chromatography is carried out on a Superdex 200 or a Sephacry S-200 column.

11. The process according to claims 3 to 10, wherein the glyco-isoform profile of the erythropoietin mixture is determined before and/or after at least one selected chromatographic step by subjecting the erythropoietin containing composition to isoelectric focusing in a gel matrix, transferring the proteins from the gel to a membrane and immuno detecting erythropoietin on the membrane.

12. The process according to claim 11, wherein the desired fractions eluted from the column are pooled.

13. The process according to any of the preceding claims, wherein in the prepared erythropoietin containing composition the proportion of the erythropoietin glyco-isoforms having up to 3 sialic acid groups per erythropoietin molecule is reduced relative to the starting erythropoietin containing composition.

14. The process according to any of the preceding claims, wherein in the prepared erythropoietin containing composition the proportion of the erythropoietin glyco-isoforms having up to 4 sialic acid groups per erythropoietin molecule is reduced relative to the starting erythropoietin containing composition.

15. The process according to any of the preceding claims, wherein in the prepared erythropoietin containing composition the proportion of the erythropoietin glyco-isoforms having up to 5 sialic acid groups per erythropoietin molecule is reduced relative to the starting erythropoietin containing composition.

16. The process according to any of the preceding claims, wherein an erythropoietin glyco-isoform mixture with a specific content of molecules with a number of sialic acid groups ranging from 6 to 14 per erythropoietin molecule is prepared.

17. The process according to any of the preceding claims, wherein an erythropoietin glyco-isoform mixture with a specific content of molecules with a number of sialic acid groups ranging from 7 to 13 per erythropoietin molecule is prepared.

18. The process according to any of the preceding claims, wherein an erythropoietin glyco-isoform mixture with a specific content of molecules with a number of sialic acid groups ranging from 8 to 13 per erythropoietin molecule is prepared.

## Patentansprüche

1. Verfahren zur Herstellung von Erythropoetin, wobei das Verfahren die Unterziehung einer Erythropoetin-haltigen Zusammensetzung den folgenden chromatographischen Schritten umfasst:
(a) Farbstoffaffinitätschromatographie
(b) Hydrophobe Chromatographie und
(c) Anionenaustauschchromatographie,
**dadurch gekennzeichnet, dass** die chromatographischen Schritte nur aus den folgenden Schritten in der angegebenen Reihenfolge bestehen (a), (b), (c) oder (a), (c), (b), jeweils optional mit
(d) Gelchromatographie.

2. Verfahren nach Anspruch 1, das zusätzlich zumindest einen Zwischenschritt des Pufferaustausches, der Filtration, der Ultrafiltration und/oder der Diafiltration umfasst.

3. Verfahren nach einem der Ansprüche 1 oder 2, worin das Erythropoetin in Form eines Glycoisoformengemisches hergestellt wird.

4. Verfahren nach Anspruch 3, worin das Glycoisoformenprofil des hergestellten Erythropoetins nach den chromatographischen Schritten relativ zur Erythropoetin-enthaltenden Ausgangszusammensetzung verändert ist.

5. Verfahren nach einem der vorangehenden Ansprüche, worin die Farbstoffaffinitätschromatographie mit einem Matrix-gebundenen Triazinfarbstoff ausgeführt wird.

6. Verfahren nach einem der vorangehenden Ansprüche, worin im Farbstoffaffinitätschromatographieschritt der pH des Elutionspuffers auf einen Bereich von 5,0 bis 9,0 eingestellt wird.

7. Verfahren nach einem der vorangehenden Ansprüche, worin im Farbstoffaffinitätschromatographieschritt der pH des Elutionspuffers auf einen Bereich von 6,0 bis 8,0 eingestellt wird.

8. Verfahren nach einem der vorangehenden Ansprüche, worin die hydrophobe Chromatographie mit einem butylierten Matrixträger ausgeführt wird.

9. Verfahren nach einem der vorangehenden Ansprüche, worin die Anionenaustauschchromatographie an einer DEAE Sepharosematrix ausgeführt wird.

10. Verfahren nach einem der vorangehenden Ansprüche, worin die Gelchromatographie auf einer Superdex 200 oder einer Sephacryl S-200 Säule ausgeführt wird.

11. Verfahren nach einem der Ansprüche 3 bis 10, worin das Glycoisoformenprofil des Erythropoetingemisches vor und/oder nach zumindest einem ausgewählten Chromatographieschritt bestimmt wird, wobei die Erythropoetin-enthaltende Zusammensetzung einer isoelektrischen Fokussierung in einer Gelmatrix unterzogen wird, die Proteine vom Gel auf eine Membran übertragen werden und das Erythropoetin auf der Membran immundetektiert wird.

12. Verfahren nach Anspruch 11, worin die gewünschten Fraktionen, die von der Säule eluieren, vereinigt werden.

13. Verfahren nach einem der vorangehenden Ansprüche, worin in der hergestellten Erythropoetin-enthaltenden Zusammensetzung der Anteil an Erythropoetin enthaltenden Glycoisoformen mit bis zu 3 Sialinsäuregruppen pro Erythropoetinmolekül relativ zur Erythropoetin-enthaltenden Ausgangszusammensetzung verringert ist.

14. Verfahren nach einem der vorangehenden Ansprüche, worin in der hergestellten Erythropoetin-enthaltenden Zusammensetzung der Anteil an Erythropoetin enthaltenden Glycoisoformen mit bis zu 4 Sialinsäuregruppen pro Erythropoetinmolekül relativ zur Erythropoetin-enthaltenden Ausgangszusammensetzung verringert ist.

15. Verfahren nach einem der vorangehenden Ansprüche, worin in der hergestellten Erythropoetin-enthaltenden Zusammensetzung der Anteil an Erythropoetin enthaltenden Glycoisoformen mit bis zu 5 Sialinsäuregruppen pro Erythropoetinmolekül relativ zur Erythropoetin-enthaltenden Ausgangszusammensetzung verringert ist.

16. Verfahren nach einem der vorangehenden Ansprüche, worin ein Erythropoetinglycoisoformengemisch mit einem spezifischen Gehalt an Molekülen mit einer Anzahl an Sialinsäuregruppen, die von 6 bis 14 pro Erythropoetinmolekül reicht, hergestellt wird.

17. Verfahren nach einem der vorangehenden Ansprüche, worin ein Erythropoetinglycoisoformengemisch mit einem spezifischen Gehalt an Molekülen mit einer Anzahl an Sialinsäuregruppen, die von 7 bis 13 pro Erythropoetinmolekül reicht, hergestellt wird.

18. Verfahren nach einem der vorangehenden Ansprüche, worin ein Erythropoetinglycoisoformengemisch mit einem spezifischen Gehalt an Molekülen mit einer Anzahl an Sialinsäuregruppen, die von 8 bis 13 pro Erythropoetinmolekül reicht, hergestellt wird.

## Revendications

1. Procédé de préparation d'érythropoïétine, lequel procédé comprend l'exposition d'une composition contenant l'érythropoïétine aux étapes chromatographiques suivantes :
(a) chromatographie par affinité du colorant,
(b) chromatographie hydrophobe et
(c) chromatographie par échange d'anions,
**caractérisé en ce que** les étapes chromatographiques sont constituées seulement des étapes suivantes dans l'ordre indiqué :
(a), (b), (c) ou (a), (c), (b), chacune avec une chromatographie sur gel (d) en option.

2. Procédé selon la revendication 1, comprenant en outre au moins une étape intermédiaire d'échange de tampon, de filtration, d'ultrafiltration et/ou de diafiltration.

3. Procédé selon la revendication 1 ou 2, dans lequel l'érythropoïétine est préparée sous la forme de mélange de glyco-isoformes.

4. Procédé selon la revendication 3, dans lequel le profil de glyco-isoformes de l'érythropoïétine préparée est changé après les étapes chromatographiques par rapport à la composition de départ contenant l'érythropoïétine.

5. Procédé selon l'une quelconque des revendications précédentes, dans lequel la chromatographie par affinité du colorant est réalisée avec un colorant triazine lié à la matrice.

6. Procédé selon l'une quelconque des revendications précédentes, dans lequel le pH du tampon d'élution est ajusté dans la plage de 5,0 à 9,0 pour l'étape chromatographique par affinité du colorant.

7. Procédé selon l'une quelconque des revendications précédentes, dans lequel le pH du tampon d'élution est ajusté dans la plage de 6,0 à 8,0 pour l'étape chromatographique par affinité du colorant.

8. Procédé selon l'une quelconque des revendications précédentes, dans lequel la chromatographie hydrophobe est réalisée avec un support matriciel butylé.

9. Procédé selon l'une quelconque des revendications précédentes, dans lequel la chromatographie par échange d'anions est réalisée sur une matrice de DEAE Sepharose.

10. Procédé selon l'une quelconque des revendications précédentes, dans lequel la chromatographie sur gel est réalisée sur une colonne de Superdex 200 ou Sephacryl S-200.

11. Procédé selon l'une des revendications 3 à 10, dans lequel le profil de glyco-isoformes du mélange d'érythropoïétine est déterminé avant et/ou après au moins une étape chromatographique choisie en soumettant la composition contenant l'érythropoïétine à une focalisation isoélectrique dans une matrice de gel, en transférant les protéines du gel vers une membrane et en immunodétectant l'érythropoïétine sur la membrane.

12. Procédé selon la revendication 11, dans lequel les fractions recherchées éluées à partir de la colonne sont regroupées.

13. Procédé selon l'une quelconque des revendications précédentes, dans lequel la proportion de glyco-isoformes ayant jusqu'à 3 groupes d'acide sialique par molécule d'érythropoïétine dans la composition préparée contenant l'érythropoïétine est réduite par rapport à la composition de départ contenant l'érythropoïétine.

14. Procédé selon l'une quelconque des revendications précédentes, dans lequel la proportion de glyco-isoformes ayant jusqu'à 4 groupes d'acide sialique par molécule d'érythropoïétine dans la composition préparée contenant l'érythropoïétine est réduite par rapport à la composition de départ contenant l'érythropoïétine.

15. Procédé selon l'une quelconque des revendications précédentes, dans lequel la proportion de glyco-isoformes ayant jusqu'à 5 groupes d'acide sialique par molécule d'érythropoïétine dans la composition préparée contenant l'érythropoïétine est réduite par rapport à la composition de départ contenant l'érythropoïétine.

16. Procédé selon l'une quelconque des revendications précédentes, dans lequel un mélange de glyco-isoformes d'érythropoïétine avec une teneur spécifique en molécules ayant un nombre de groupes d'acide sialique allant de 6 à 14 par molécule d'érythropoïétine est préparé.

17. Procédé selon l'une quelconque des revendications précédentes, dans lequel un mélange de glyco-isoformes d'érythropoïétine avec une teneur spécifique en molécules ayant un nombre de groupes d'acide sialique allant de 7 à 13 par molécule d'érythropoïétine est préparé.

18. Procédé selon l'une quelconque des revendications précédentes, dans lequel un mélange de glyco-isoformes d'érythropoïétine avec une teneur spécifique en molécules ayant un nombre de groupes d'acide sialique allant de 8 à 13 par molécule d'érythropoïétine est préparé.
